# EUROPEAN PATENT SPECIFICATION

(11) **EP 0 741 588 B1**
(45) Date of publication and mention of the grant of the patent: **18.11.1998**
(21) Application number: 95907917.9
(22) Date of filing: 19.01.1995
(51) Int. Cl.: A61M 11/00

(54) **METHOD TO PRODUCE AN AEROSOL**
VERFAHREN ZUR AEROSOLHERSTELLUNG
PROCEDE DE PRODUCTION D'UN AEROSOL

(30) Priority: 26.01.1994 SE 9400241
(43) Date of publication of application: 13.11.1996
(73) Proprietor: AGA AKTIEBOLAG, 181 81 Lidingö (SE)
(72) Inventor: HAMMARLUND, Nils, S-191 71 Sollentuna (SE)
(74) Representative: Axelsson, Rolf
(86) International application number: SE9500044
(87) International publication number: WO9520411

(56) References cited:
- WO-A-85/02777
- WO-A-94/09842
- US-A- 5 027 806
- US-A- 5 228 434

## Description

The present invention relates to a method of bringing a medicament into a form in which it can be inhaled, there being obtained an aerosol which when inhaled penetrates further down into the airways and results in more effective use of the medicament.

In the case of respiratory tract infections, such as asthma and chronic bronchitis, it is highly beneficial to be able to deliver a medicament via inhalation, both with respect to maintenance treatment and in respect to acute deterioration of the illness. The medicament is conveniently administered with the aid of a nebulizer, which is normally driven with compressed air or with pure oxygen gas.

One problem experienced in inhalation treatment processes is that of being able to administer the medicament to the location where it will do most good. When using traditional nebulizing equipment, a large part of the administered dosage will land up in the oral cavity and pharynx and only a small part of the dosage will arrive in the bronchial tree. A highly effective nebulizer has recently been described in EP-B1-0191018. Improved peripheral deposition of the nebulized medicament is obtained with this nebulizer, and therewith a better effect, less risk of side effects, lower medicament consumption and shorter treatment times.

US-A-5228434 discloses the use of stable xenon as the anesthetizing agent in a gas mixture delivered to a patient. Xenon has a high density and in order to obtain an anesthetic gas mixture of lower density helium is added to the mixture. However, this specification does not aim at obtaining an improved method of converting a medicament into an aerosol which is more effectively deposited in the airways than the aerosols hitherto used.

Studies made on people suffering from mild to moderate asthma have shown that these deposit inhaled particles more centrally in the bronchial tree than do healthy people. This may be due to a stronger tendency towards turbulence in the airways of asthmatics, for instance, which may, in turn, be because the airways of such people are narrow or partially blocked, as in the case with cystic fibrosis, for instance.

An object of the present invention is to improve further the peripheral deposition of medicaments administered to the airways or respiratory tracks of a patient and therewith reduce the necessary effective medicament dosage, or to shorten the necessary treatment time, or a combination of these benefits. From a medical aspect, it is important that the effect of treatment is achieved rapidly. More effective deposition and lower local medicament dosages also reduce the risk of side effects.

Another object of the invention is to provide a medicament preparation in the form of an aerosol which consists in liquid particles that are comprised of or include a medicament and a gas mixture which is able to transport the medicament-containing liquid further down into the airways or respiratory tracts of a patient than those gas flows hitherto used.

Still a further object of the invention is to provide the use of a gas mixture for producing an aerosol, said aerosol being comprised of liquid particles which include medicaments, and a carrier gas flow which is able to transport the medicament-containing liquid further down into the airways, to the alveoli than hitherto used gas flows.

According to the present invention, the peripheral deposition of medicaments delivered to the airways or respiratory tracts is improved when the medicament is a liquid or is dissolved or dispersed in a liquid, the liquid is converted to liquid particles and collected in a carrier gas which contains helium and/or neon. The carrier gas is preferably a mixture of helium and/or neon and oxygen, particularly helium and oxygen. The helium and/or neon concentration will advantageously constitute at least 70 percent by volume of the carrier gas. The liquid used will be compatible with the airways, and may be water for instance. Advantageous embodiments of the invention will be apparent from the depending Claims.

The advantage of using helium and/or neon as a carrier gas for liquid particles which consist in or include a medicament is that at normal pressure and temperatures helium in particular, but also neon, has a lower density and viscosity than other gaseous substances, for instance air. This property is utilized for passing a medicament further down into the finest airways, the alveoli, with lower turbulence.

The present invention will now be described in more detail with reference to the accompanying drawing, in which
Fig. 1 is a schematic cross-sectional view of a nebulizer in which liquid particles are produced with the aid of a gas stream; and
Fig. 2 is a schematic cross-sectional view of a nebulizer in which liquid particles are produced ultrasonically.

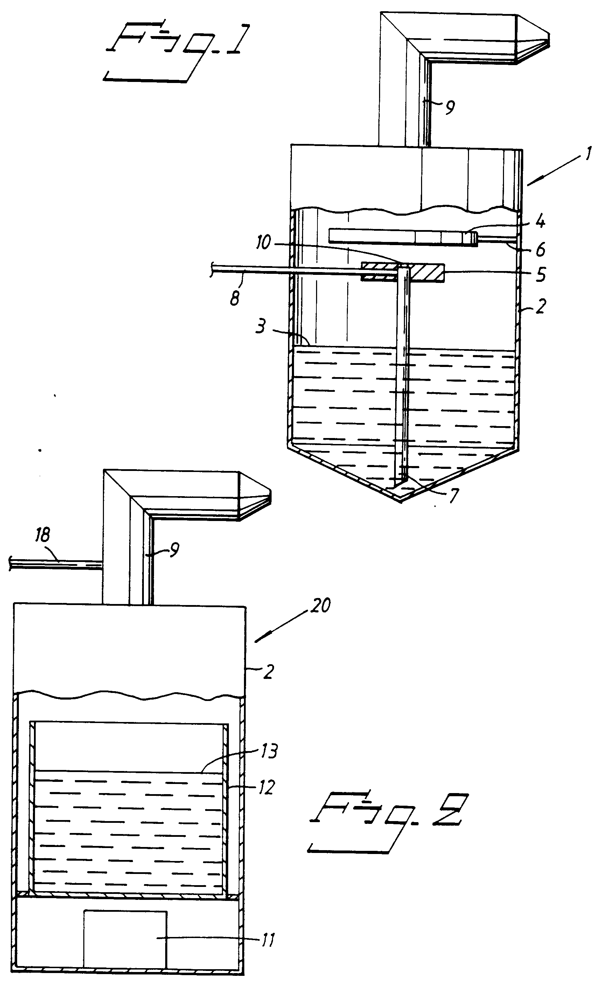

Fig. 1 illustrates a nebulizer 1 which includes a container 2 containing a liquid 3. The liquid may be a medicament or may consist in a medicament solution or a medicament dispersion, for instance a medicament against asthma. The nebulizer 1 also includes two mutually spaced and mutually parallel discs 4, 5, of which the bottom disc 5 is connected with the liquid 3 through a conduit 7. The conduit 7 terminates in a small opening 10 in the upper surface of the disc 5. The conduit 7 is connected with a propellant gas conduit immediately beneath the opening 10. This propellant gas conduit is connected to a gas source, normally air or oxygen. The nebulizer also includes an outlet 9 for the aerosol produced.

The medicament-containing liquid 3 is present in the container 2 when, taking the nebulizer 1 into use. The propellant gas is caused to flow through the conduit 8 to the conduit 7 and thereafter through the opening 10. The gas therewith draws liquid 3 from the container 2 by suction, this liquid accompanying the gas flow onto the upper disc 4, where the liquid is spread radially and forms small liquid droplets at the disc edges, these liquid droplets being transported towards the outlet 9 by the gas flow.

Fig. 2 illustrates a nebulizer 20 in which liquid droplets are produced ultrasonically. The nebulizer includes a container 12 which contains a liquid 13 in which a medicament is dissolved or dispersed. An ultrasound source 11 is mounted beneath the container 12. A gas conduit 18 connected to a source of gas under pressure opens out into an outlet 9. The gas used is normally air or oxygen.

The container 12 is filled with liquid when using the nebulizer 20. The ultrasound source is connected to an energy source in a manner not shown, to produce small liquid particles across the surface of the liquid. The gas flowing through the conduit 18 entrains these liquid particles through the outlet opening 9.

According to another embodiment of the nebulizer similar to the Fig. 1 embodiment, the upper disc 4 is replaced with a rapidly rotating disc. The liquid leaving the opening in the bottom disc impinges on the rotating disc and is divided thereby into small particles.

In accordance with the invention, there is used for producing the present medicament-containing aerosol a gas or a gas mixture whose components have a low molecular weight. The gas is characterized by containing helium and/or neon. The gas will-also conveniently contain oxygen, in addition to helium and/or neon. The preferred light gas is helium. The oxygen content should not be less than 15 percent by weight, and is conveniently about 20 percent or higher. In a preferred embodiment, the helium and/or neon content is at lowest 70 percent by volume.

Suitable medicaments for conversion to an aerosol form are, for instance, BRICANYL® and ADRENALIN marketed by Draco; VENTOLINE and BECOTIDE® marketed by Glaxo; and, LOMUDAL® marketed by Fisons.

## Claims

1. A method of converting a medicament to a form usable for administration of the medicament to the airways or respiratory tracts of mammals, wherein a liquid which consists in or includes a medicament is transformed to an aerosol which contains small liquid particles with the aid of a gas stream or with the aid of other means, and wherein the liquid particles are collected in the gas stream or in a gas stream respectively, **characterized** by collecting the liquid particles in a helium-containing and/or a neon-containing gas.

2. A method according to Claim 1, **characterized** in that the gas is a mixture of helium and/or neon and oxygen.

3. A method according to Claim 1 or Claim 2, **characterized** in that the gas contains at lowest 70 percent by volume helium and/or neon.

4. The use of a helium-containing and/or neon-containing gas mixture to produce an aerosol which includes liquid particles that consist in or include medicament, and a carrier gas.

5. The use according to Claim 4, **characterized** in that the liquid particles are produced with the aid of the carrier gas.

6. The use according to Claim 4 or Claim 5, **characterized** in that the gas is a mixture that contains helium and/or neon and oxygen.

7. The use according to Claim 6, **characterized** in that the helium-content and/or the neon-content of the gas is at lowest 70 percent by volume.

8. A medicament preparation in the form of an aerosol, **characterized** in that the aerosol is comprised of liquid particles which consist in or include medicament, and a gas mixture containing helium and/or neon.

9. A preparation according to Claim 8, **characterized** in that the gas mixture is a mixture containing helium and/or neon and oxygen.

10. A preparation according to Claim 8 or Claim 9, **characterized** in that the gas mixture contains at lowest 70 percent by volume helium and/or neon.

## Patentansprüche

1. Verfahren zum Umwandeln eines Arzneimittels in eine Form, die für die Anwendung des Arzneimittels bei den Luftwegen oder Atemwegen von Säugetieren verwendbar ist, wobei eine Flüssigkeit, die aus einem Arzneimittel besteht oder ein solches einschließt in ein Aerosol, das kleine Flüssigkeitsteilchen enthält, mit Hilfe eines Gasstroms oder mit Hilfe anderer Mittel umgewandelt wird und bei dem die Flüssigkeitsteilchen in dem Gasstrom oder in einem Gasstrom gesammelt werden, dadurch gekennzeichnet, daß die Flüssigkeitsteilchen in einem Helium enthaltenden und/oder einem Neon enthaltenden Gas gesammelt werden.

2. Verfahren nach Anspruch 1, dadurch gekennzeichnet, daß das Gas eine Mischung von Helium und/oder Neon und Sauerstoff ist.

3. Verfahren nach Anspruch 1 oder 2, dadurch gekennzeichnet, daß das Gas wenigstens 70 Volumenprozent Helium und/oder Neon enthält.

4. Verwendung einer Helium enthaltenden und/oder Neon enthaltenden Gasmischung zum Erzeugen eines Aerosols, das Flüssigkeitsteilchen, die aus einem Arzneimittel bestehen oder ein solches enthalten, und ein Trägergas einschließt.

5. Verwendung nach Anspruch 4, dadurch gekennzeichnet, daß die Flüssigkeitsteilchen mit Hilfe des Trägergases erzeugt werden.

6. Verwendung nach Anspruch 4 oder 5, dadurch gekennzeichnet, daß das Gas eine Mischung ist, die Helium und/oder Neon und Sauerstoff enthält.

7. Verwendung nach Anspruch 6, dadurch gekennzeichnet, daß der Heliumgehalt und/oder der Neongehalt des Gases mindestens 70 Volumenprozent beträgt.

8. Arzneimittelzubereitung in Form eines Aerosols, dadurch gekennzeichnet, daß das Aerosol aus Flüssigkeitsteilchen, die aus einem Arzneimittel bestehen oder ein solches enthalten, und einer Gasmischung besteht, die Helium und/oder Neon enthält.

9. Zubereitung nach Anspruch 8, dadurch gekennzeichnet, daß die Gasmischung eine Mischung ist, die Helium und/oder Neon und Sauerstoff enthält.

10. Zubereitung nach Anspruch 8 oder 9, dadurch gekennzeichnet, daß die Gasmischung mindestens 70 Volumenprozent Helium und/oder Neon enthält.

## Revendications

1. Un procédé pour convertir un médicament en une forme utilisable pour l'administration du médicament par les conduits ventilatoires ou voies respiratoires des mammifères, dans lequel un liquide constitué d'un ou comportant un médicament est transformé en un aérosol qui renferme de petites particules de liquide à l'aide d'un courant gazeux ou bien à l'aide d'autres moyens, et dans lequel les particules de liquide sont recueillies dans le courant gazeux ou bien dans un courant de gaz, respectivement, caractérisé en ce que l'on recueille les particules de liquide dans un gaz renfermant de l'hélium et/ou un gaz renfermant du néon.

2. Un procédé selon la revendication 1, caractérisé en ce que le gaz est un mélange d'hélium et/ou de néon et d'oxygène.

3. Un procédé selon la revendication 1 ou la revendication 2, caractérisé en ce que le gaz renferme au moins 70 pour-cent en volume d'hélium et/ou de néon.

4. L'utilisation d'un mélange gazeux renfermant de l'hélium et/ou renfermant du néon afin de préparer un aérosol qui comporte des particules liquides qui sont constituées d'un ou comportent un médicament, et un gaz support.

5. L'utilisation selon la revendication 4, caractérisée en ce que les particules de liquide sont préparées à l'aide du gaz support.

6. L'utilisation selon la revendication 4 ou la revendication 5, caractérisée en ce que le gaz est un mélange qui renferme de l'hélium et/ou du néon et de l'oxygène.

7. L'utilisation selon la revendication 6, caractérisée en ce que la teneur en hélium et/ou la teneur en néon du gaz est d'au moins 70 pour-cent en volume.

8. Une préparation de médicament sous la forme d'un aérosol, caractérisée en ce que l'aérosol est constitué de particules de liquide qui sont constituées d'un ou comportent un médicament et un mélange gazeux renfermant de l'hélium et/ou du néon.

9. Une préparation selon la revendication 8, caractérisée en ce que le mélange gazeux est un mélange renfermant de l'hélium et/ou du néon et de l'oxygène.

10. Une préparation selon la revendication 8 ou la revendication 9, caractérisée en ce que le mélange gazeux renferme au moins 70 pour-cent en volume d'hélium et/ou de néon.
